# EUROPÄISCHE PATENTSCHRIFT

(11) **EP 1 419 148 B1**
(45) Veröffentlichungstag und Bekanntmachung des Hinweises auf die Patenterteilung: **13.09.2006**
(21) Anmeldenummer: 02730198.5
(22) Anmeldetag: 24.04.2002
(51) Int. Cl.: C07D 333/20, C07D 307/52, C07D 277/28, C07D 333/28, C07D 333/22, A61Q 5/10

(54) **M-AMINOPHENOL-DERIVATE UND DIESE VERBINDUNGEN ENTHALTENDE FÄRBEMITTEL**
M-AMINOPHENOL DERIVATIVES AND COLOURING AGENTS CONTAINING THEM
DERIVES DE M-AMINOPHENOL ET COLORANTS CONTENANT CES COMPOSES

(30) Priorität: 25.08.2001 DE 10141723
(43) Veröffentlichungstag der Anmeldung: 19.05.2004
(73) Patentinhaber: Wella Aktiengesellschaft, 64274 Darmstadt (DE)
(72) Erfinder: PASQUIER, Cécile, CH-1723 Marly (CH); WYSS, Patrick, CH-1212 Grand-Lancy (CH); BRAUN, Hans-Jürgen, CH-3182 Überstorf (CH)
(74) Vertreter: Bockhorst, Matthias
(86) Internationale Anmeldenummer: PCT/EP2002/004495
(87) Internationale Veröffentlichungsnummer: WO 2003/018571

(56) Entgegenhaltungen:
- EP-A- 0 963 982
- WO-A-01/85683
- DE-A- 2 118 494
- US-A- 5 019 130
- VOEGTLI ET AL.: "168. Ueber die Eigenschaften einiger 4-(o-Oxyphenyl)-thiazole" HELV. CHIM. ACTA, Bd. 33, 1950, XP001098779

## Beschreibung

Die vorliegende Erfindung betrifft neue in 6-Stellung substituierte 3-Aminophenol-Derivate sowie diese Verbindungen enthaltende Mittel zum Färben von Keratinfasem, insbesondere menschlichen Haaren.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 1,4-Diaminobenzol und 4,5-Diamino-1-(2-hydroxyethyl)-pyrazol eingesetzt, während als Kupplersubstanzen beispielsweise Resorcin, 2-Methyl-resorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen sind.

Weitere kupplersubstanzen sind aus der US 5 019 130 bekannt.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, dass durch Kombination geeigneter Entwicklersubstanzen und Kupplersubstanzen eine breite Palette verschiedener Farbnuancen erzeugt werden kann.

Obwohl bereits eine Vielzahl von Kupplersubstanzen bekannt, ist es mit den derzeit bekannten Färbemitteln nicht möglich, die an ein Färbemittel gestellten Anforderungen in jeder Hinsicht zu erfüllen. Es besteht daher weiterhin ein Bedürfnis nach neuen Kupplersubstanzen, welche die vorgenannten Anforderung in besonderem Masse erfüllen.

Aus Voegtli et al. In Helv. Chim. Acta Vol. 33 (1950), Seiten 1297-1303 sind 4-(o-Oxyphenyl)-thiazole und deren Verwendung als bakteriostatisch wirksame Verbindungen bekannt.

Es wurde nunmehr gefunden, dass bestimmte 3-Aminophenol-Derivate gemäß der allgemeinen Formel (I) die an Kupplersubstanzen gestellten Anforderungen in besonders hohem Masse erfüllen und mit den meisten bekannten Entwicklersubstanzen farbstarke, außerordentlich lichtechte und waschechte Farbnuancen ergeben.

Gegenstand der vorliegenden Erfindung ist daher ein Mittel zur Färbung von Keratinfasern, wie zum Beispiel Wolle, Pelzen, Federn oder Haaren und insbesondere menschlichem Haaren, auf der Basis einer Entwicklersubstanz-Kupplersubstanz-Kombination, welches dadurch gekennzeichnet ist, dass es mindestens ein 3-Aminophenol-Derivat der Formel (I) oder dessen physiologisch verträglichen wasserlösliche Salze enthält, 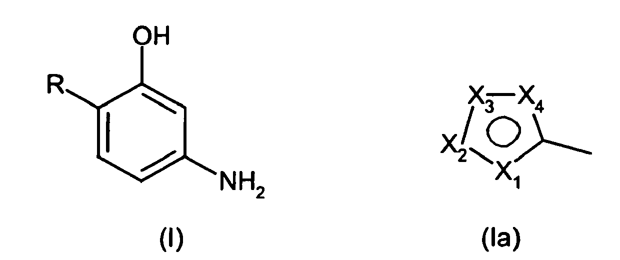 mit **R** gleich einem aromatischen, heterozyklischen 5-Ring der Formel (la); wobei **X1, X2, X3** und **X4** unabhängig voneinander gleich Schwefel, Stickstoff, N-R1, Sauerstoff, C-R2, C-R3, C-R4 oder C-R5 sind, unter der Bedingung, dass mindestens einer und höchstens drei der Reste **X1** bis **X4** gleich Stickstoff, N-R1, Schwefel oder Sauerstoff sind und wenn es mehrere Heteroatome gibt höchstens einer der Reste **X1** bis **X4** gleich Schwefel, N-R1 oder Sauerstoff ist;
**R1** gleich Wasserstoff, einer C₁-C₆-Alkylgruppe, einer Phenylgruppe, einer (C₂-C₄)-Hydroxyalkylgruppe oder einerAcetylgruppe ist;
**R2, R3, R4** und **R5** unabhängig voneinander Wasserstoff, ein Halogenatom (F, Cl, Br, J), eine Cyanogruppe, eine C₁-C₆-Alkylgruppe, eine C₁-C₄-Alkoxygruppe, eine C₁-C₄-Alkylthioethergruppe, eine Mercaptogruppe, eine Nitrogruppe, eine Aminogruppe, eine C₁-C₄-Alkylaminogruppe, eine Di(C₁-C₄)alkylaminogruppe, eine Hydroxy(C₂-C₄)alkylaminogruppe, eine Di(hydroxy(C₂-C₄)alkyl)aminogruppe, eine (Dihydroxy(C₃-C₄)alkyl)-aminogruppe, eine Trifluormethangruppe, eine -C(O)CH₃-Gruppe, eine -C(O)CF₃-Gruppe, eine -C(O)H-Gruppe, eine -Si(CH₃)₃-Gruppe, eine (C₁-C₄)-Hydroxyalkylgruppe oder eine (C₂-C₄)-Dihydroxyalkylgruppe darstellen.

Als Verbindungen der Formel (I) können beispielweise genannt werden: 5-amino-2-(3-thienyl)phenol, 5-amino-2-(3-furyl)phenol, 5-amino-2-(pyrrol-3-yl)phenol, 5-amino-2-(1-methyl-1H-pyrrol-3-yl)phenol, 5-amino-2-(1,3-thiazol-2-yl)phenol, 5-amino-2-(1,3-thiazol-5-yl)phenol, 5-amino-2-(2-thienyl)phenol, 5-amino-2-(2-furyl)phenol, 5-amino-2-(pyrrol-2-yl)phenol, 5-amino-2-(1-methyl-1H-pyrrol-2-yl)phenol, 5-amino-2-(2-chlor-3-thienyl)-phenol, 5-amino-2-(2-methyl-3-thienyl)phenol, 5-amino-2-(2-nitro-3-thienyl)phenol, 5-amino-2-(2-amino-3-thienyl)phenol, 5-amino-2-(2-acetyl-3-thienyl)phenol, 5-amino-2-(2-formyl-3-thienyl)phenol, 5-amino-2-(4-chlor-3-thienyl)phenol, 5-amino-2-(4-methyl-3-thienyl)phenol, 5-amino-2-(4-nitro-3-thienyl)phenol, 5-amino-2-(4-amino-3-thienyl)phenol, 5-amino-2-(4-acetyl-3-thienyl)phenol, 5-amino-2-(4-formyl-3-thienyl)phenol, 5-amino-2-(5-chlor-3-thienyl)phenol, 5-amino-2-(5-methyl-3-thienyl)phenol, 5-amino-2-(5-nitro-3-thienyl)phenol, 5-amino-2-(5-acetyl-3-thienyl)phenol, 5-amino-2-(5-amino-3-thienyl)phenol, 5-amino-2-(5-formyl-3-thienyl)phenol, 5-amino-2-(5-formyl-3-furyl)phenol, 5-amino-2-(3-chlor-2-thienyl)phenol, 5-amino-2-(3-methyl-2-thienyl)phenol, 5-amino-2-(3-nitro-2-thienyl)phenol, 5-amino-2-(3-amino-2-thienyl)phenol, 5-amino-2-(3-acetyl-2-thienyl)-phenol, 5-amino-2-(3-formyl-2-thienyl)phenol, 5-amino-2-(4-chlor-2-thienyl)phenol, 5-amino-2-(4-methyl-2-thienyl)phenol, 5-amino-2-(4-nitro-2-thienyl)phenol, 5-amino-2-(4-amino-2-thienyl)phenol, 5-amino-2-(4-acetyl-2-thienyl)phenol, 5-amino-2-(4-formyl-2-thienyl)phenol, 5-amino-2-(5-chlor-2-thienyl)phenol, 5-amino-2-(5-methyl-2-thienyl)phenol, 5-amino-2-(5-nitro-2-thienyl)phenol, 5-amino-2-(5-amino-2-thienyl)phenol, 5-amino-2-(5-acetyl-2-thienyl)phenol, 5-amino-2-(5-formyl-2-thienyl)phenol, 5-amino-2-(5-formyl-2-furyl)phenol, 5-amino-2-(5-nitro-1,3-thiazol-2-yl)phenol, 5-amino-2-(5-amino-1,3-thiazol-2-yl)phenol, 5-amino-2-(2-nitro-1,3-thiazol-5-yl)phenol, 5-amino-2-(2-amino-1,3-thiazol-5-yl)phenol, 5-amino-2-(3,5-dimethyl-1H-pyrazol-4-yl)phenol und 5-amino-2-(5-nitro-4H-1,2,4-triazol-3-yl)phenol sowie deren physiologisch verträglichen wasserlösliche Salze.

Bevorzugt sind Verbindungen der Formel (I) in denen gilt:
(i) **X2** ist gleich Schwefel, und **X1, X3** und **X4** sind gleich C-R2, C-R4 und C-R5 oder (ii) **X1** ist gleich Schwefel und **X2**, **X3** und **X4** sind gleich C-R3, C-R4 und C-R5.

Besonders bevorzugt sind die folgenden Verbindungen der Formel (I): 5-amino-2-(3-thienyl)phenol, 5-amino-2-(2-chlor-3-thienyl)phenol und 5-amino-2-(5-chlor-2-thienyl)phenol sowie deren physiologisch verträglichen wasserlösliche Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die 3-Aminophenol-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen kommen vorzugsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)-amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methylethyl)-1 H-pyrazol, 4,5-Diamino-1-[(4-methylphenyl)methyl]-1 H-pyrazol, 1-[(4-Chlorphenyl)methyl]-4,5-diamino-1 H-pyrazol, 4,5-Diamino-1-methyl-1 H-pyrazol, 2-Amino-phenol, 2-Amino-6-methyl-phenol, 2-Amino-5-methyl-phenol und 1,2,4-Trihydroxy-benzol in Betracht.

Weiterhin kann das erfindungsgemäße Färbemittel zusätzlich zu den Verbindungen der Formel (I) noch weitere bekannte Kupplersubstanzen, beispielsweise N-(3-Dimethylamino-phenyl)-harnstoff, 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbkomponenten, wie zum Beispiel 6-Amino-2-methylphenol und 2-Amino-5-methylphenol, sowie ferner übliche synthetische oder natürliche direktziehende Farbstoffe, beispielsweise Pflanzenfarbstoffe oder synthetische direktziehende Farbstoffe aus der Gruppe der sauren oder basischen Farbstoffe, der Triphenylmethanfarbstoffe, der aromatischen Nitrofarbstoffe, der Azofarbstoffe und der Dispersionsfarbstoffe, enthalten. Die erfindungsgemäßen Färbemittel können diese Farbkomponenten in einer Menge von etwa 0,1 bis 4 Gewichtsprozent enthalten.

Selbstverständlich können die zusätzlichen Kupplersubstanzen sowie die Entwicklersubstanzen und die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch Aminosäuren und/oder organische Amine, zum Beispiel Monoethanolamin und Triethanolamin, sowie anorganische Basen, wie zum Beispiel Natriumhydroxid und Kaliumhydroxid Verwendung finden. Für eine pH-Einstellung im sauren Bereich kommen anorganische oder organische Säuren, zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an 3-Aminophenol-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, dass dieses Mittel insbesondere auch eine Anfärbung von ergrauten, chemisch nicht vorgeschädigten Haaren problemlos und mit guter Deckkraft ermöglicht.

Die Herstellung der erfindungsgemäßen Aminophenol-Derivate der Formel (I) kann unter Verwendung von literaturbekannten Syntheseverfahren erfolgen, beispielsweise
**a)** durch eine Tetrakis(triphenylphospin)palladium(0) katalysierte Kupplung eines geeigneten substituierten 3-Aminophenol-borsäurederivates der Formel (IIa) mit einer halogensubstituierten aromatischen Verbindung der Formel (IIIa) und anschließende Abspaltung der für die Kupplungsreaktion erforderlichen Schutzgruppen, 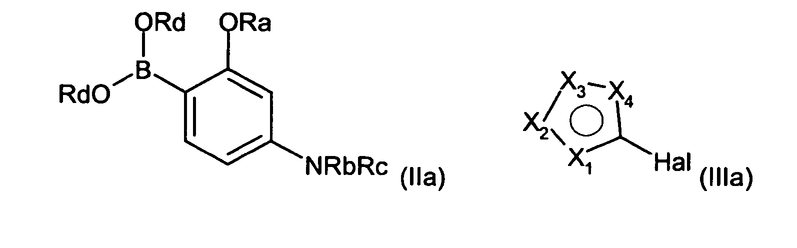 oder
**b)** durch eine Tetrakis(triphenylphospin)palladium(0) katalysierte Kupplung eines halogensubstituierten 3-Aminophenol der Formel (IIb) mit einem Borsäurederivat der Formel (IIIb) und anschließende Abspaltung 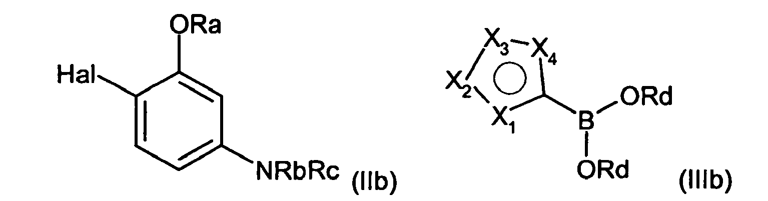
der für die Kupplungsreaktion erforderlichen Schutzgruppen; wobei die Restgruppen in den Formeln (IIa), (IIb), (IIIa) und (IIIb) die folgende Bedeutung haben:
**Ra** steht für eine Schutzgruppe, wie sie zum Beispiel in dem Kapitel "Protective Groups" in Organic Synthesis, Kapitel 3, Wiley Interscience, 1991 beschrieben wird;
**Rb** und **Rc** stehen unabhängig voneinander für eine Schutzgruppe, wie sie zum Beispiel in dem Kapitel "Protective Groups" in Organic Synthesis, Kapitel 7, Wiley Interscience, 1991 beschrieben wird, oder Wasserstoff; **Rd** ist gleich Wasserstoff oder die beiden Rd-Reste bilden gemeinsam mit der O-B-O-Gruppe einen unsubstituierten oder substituierten fünfgliedrigen oder sechsgliedrigen cycloaliphatischen Ring;
**Hal** ist gleich F, Cl, Br oder J; und
**X1, X2, X3** und **X4** haben die in der Formel (I) angegebene Bedeutung.

Die 3-Aminophenol-Derivate der Formel (I) sind gut in Wasser löslich und ermöglichen Färbungen mit ausgezeichneter Farbintensität und Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der hier beschriebenen Oxidationsfärbemittel, auf.

Ein weiterer Gegenstand der vorliegenden Erfindung sind neue 3-Aminophenol-Derivate der Formel (I), oder deren physiologisch verträglichen wasserlösliche Salze, bei denen (i) **X2** gleich Schwefel ist, und **X1, X3** und **X4** gleich C-R2, C-R4 und C-R5 sind oder (ii) **X1** gleich Schwefel ist und **X2, X3** und **X4** gleich C-R3, C-R4 und C-R5 sind, und die Reste **R2** bis **R4** die vorgenannte Bedeutung haben.

Gegenstand der vorliegenden Erfindung sind ebenfalls neue 3-Aminophenol-Derivate der Formel (I), welche ausgewählt sind aus 5-amino-2-(3-thienyl)phenol, 5-amino-2-(3-furyl)phenol, 5-amino-2-(pyrrol-3-yl)phenol, 5-amino-2-(1-methyl-1H-pyrrol-3-yl)phenol, 5-amino-2-(1,3-thiazol-2-yl)phenol, 5-amino-2-(1,3-thiazol-5-yl)phenol, 5-amino-2-(2-thienyl)phenol, 5-amino-2-(2-furyl)-phenol, 5-amino-2-(pyrrol-2-yl)phenol, 5-amino-2-(1-methyl-1 H-pyrrol-2-yl)phenol, 5-amino-2-(2-chlor-3-thienyl)-phenol, 5-amino-2-(2-methyl-3-thienyl)phenol, 5-amino-2-(2-nitro-3-thienyl)phenol, 5-amino-2-(2-amino-3-thienyl)phenol, 5-amino-2-(2-acetyl-3-thienyl)-phenol, 5-amino-2-(2-formyl-3-thienyl)phenol, 5-amino-2-(4-chlor-3-thienyl)phenol, 5-amino-2-(4-methyl-3-thienyl)phenol, 5-amino-2-(4-nitro-3-thienyl)phenol, 5-amino-2-(4-amino-3-thienyl)phenol, 5-amino-2-(4-acetyl-3-thienyl)phenol, 5-amino-2-(4-formyl-3-thienyl)phenol, 5-amino-2-(5-chlor-3-thienyl)phenol, 5-amino-2-(5-methyl-3-thienyl)phenol, 5-amino-2-(5-nitro-3-thienyl)phenol, 5-amino-2-(5-acetyl-3-thienyl)phenol, 5-amino-2-(5-amino-3-thienyl)-phenol, 5-amino-2-(5-formyl-3-thienyl)phenol, 5-amino-2-(5-formyl-3-furyl)phenol, 5-amino-2-(3-chlor-2-thienyl)phenol, 5-amino-2-(3-methyl-2-thienyl)phenol, 5-amino-2-(3-nitro-2-thienyl)phenol, 5-amino-2-(3-amino-2-thienyl)phenol, 5-amino-2-(3-acetyl-2-thienyl)-phenol, 5-amino-2-(3-formyl-2-thienyl)phenol, 5-amino-2-(4-chlor-2-thienyl)phenol, 5-amino-2-(4-methyl-2-thienyl)phenol, 5-amino-2-(4-nitro-2-thienyl)phenol, 5-amino-2-(4-amino-2-thienyl)phenol, 5-amino-2-(4-acetyl-2-thienyl)phenol, 5-amino-2-(4-formyl-2-thienyl)phenol, 5-amino-2-(5-chlor-2-thienyl)phenol, 5-amino-2-(5-methyl-2-thienyl)phenol, 5-amino-2-(5-nitro-2-thienyl)phenol, 5-amino-2-(5-amino-2-thienyl)phenol, 5-amino-2-(5-acetyl-2-thienyl)-phenol, 5-amino-2-(5-formyl-2-thienyl)phenol, 5-amino-2-(5-formyl-2-furyl)phenol, 5-amino-2-(5-nitro-1,3-thiazol-2-yl)phenol, 5-amino-2-(5-amino-1,3-thiazol-2-yl)phenol, 5-amino-2-(2-nitro-1,3-thiazol-5-yl)phenol, 5-amino-2-(2-amino-1,3-thiazol-5-yl)phenol, 5-amino-2-(3,5-dimethyl-1 H-pyrazol-4-yl)phenol und 5-amino-2-(5-nitro-4H-1,2,4-triazol-3-yl)phenol sowie deren physiologisch verträglichen wasserlöslichen Salzen.

Besonders bevorzugt sind das 5-amino-2-(3-thienyl)phenol, 5-amino-2-(2-chlor-3-thienyl)phenol und 5-amino-2-(5-chlor-2-thienyl)phenol sowie deren physiologisch verträglichen Salzen.

Die nachfolgenden Beispiele sollen den Gegenstand der Erfindung näher erläutern, ohne ihn darauf zu beschränken.

### Beispiele

### Beispiele 1 bis 9: Synthese von 3-Aminophenol-Derivaten der allgemeinen Formel (I) (Allgemeine Synthesevorschrift)

### A. Synthese von 3-Ethoxymethoxyphenylamin

Zu einer Lösung von 20 g (183,3 mmol) 3-Aminophenol in 450 ml getrocknetem Acetonitril gibt man bei 0 °C portionsweise 12 g (274,9 mmol) einer Natriumhydrid-Dispersion (55% in Öl). Das Gemisch wird anschließend 3 Stunden lang bei 0 °C gerührt. Dann gibt man tropfenweise eine Lösung von 25 g (210,8 mmol) Chlormethylethylether in 30 ml Acetonitril hinzu und rührt das Gemisch über Nacht bei Raum Temperatur. Anschließend wird das Reaktionsgemisch filtriert. Der Filtrationsrückstand wird mit wenig Aceton gewaschen und das Filtrat eingeengt. Es werden 32,3 g 3-Ethoxymethoxyphenylamin erhalten. Das erhaltene Rohprodukt wird ohne weitere Reinigung in der nächsten Stufe eingesetzt.
¹H-NMR (300 MHz, DMSO): δ= 6,89 (t, 1 H, H5); 6,24 (s, 1 H, H2); 6,22 (d, 1 H); 6,16 (d, 1 H); 5,14 (s; 2H, NH2); 5,11 (s, 2H, OCH2); 3,75 (q, 2H, CH₂); 1,13 (t, 3H, CH₃).

### B. Synthese von N-(3-Ethoxymethoxyphenyl)-carbaminsäure-tert.-butyl ester

30 g (180 mmol) 3-Ethoxymethoxyphenylamin aus Stufe A und 44,4 g (203 mol) Di-tert.-butyl-dicarbonat werden in einer Mischung von 140 ml 2N Natriumhydroxid und 200 ml Dichlormethan gelöst und 24 Stunden lang bei Raumtemperatur gerührt. Anschließend wird die organische Phase abgetrennt, mit einer gesättigten wässrigen Kochsalzlösung bis zu einem neutralem pH-Wert gewaschen, über MgSO₄ getrocknet, filtriert und eingeengt. Das erhaltene Rohprodukt wird an Kieselgel mit Hexan/Essigsäureethylester (8:1) gereinigt.
Es werden 18 g (42 % der Theorie bezogen auf das in Stufe A eingesetzte 3-Aminophenol) N-(3-Ethoxy-methoxyphenyl)-carbaminsäure-tert-butylester als gelbes Öl erhalten.
¹H-NMR (300 MHz, DMSO): δ = 9,33 (s, 1H, NH); 7,20 (s, 1H, H2); 7,14 (t, J=8,0, 1H, H5); 7,05 (d, J=8,0, 1H, H3), 6,63 (d, J=8,0, 1H, H6), 5,17 (s, 2H, OCH2), 3,64 (q, J=7,1, 2H, CH2), 1,49 (s, 9H, tert-butyl), 1,13 (t, J=7,1, 3H, CH3).
CHN-Analyse:

| (C₁₄H₂₁NO₄) | | % C | % H | % N |
|---|---|---|---|---|
| | berechnet: | 62,90 | 7,92 | 5,24 |
| | gefunden: | 62,60 | 8,04 | 4,97 |

### C. Synthese von N-(4-Brom-3-ethoxymethoxy-phenyl)-carbaminsäure-tert-butylester

13,9 g (52 mmol)) N-(3-Ethoxymethoxyphenyl)-carbaminsäure-tert-butylester aus Stufe B und 10,2 g (57,2 mmol) N-Bromsuccinimid werden unter Stickstoff in 400 ml 1,2-Dimethoxyethan gelöst und 3 Stunden lang bei Raumtemperatur gerührt. Anschließend wird die Reaktionsmischung auf 1000 ml Eis/Wasser gegossen und mit Essigsäureethylester extrahiert. Die organische Phase wird mit einer gesättigten wässrigen Kochsalzlösung gewaschen, über Magnesiumsulfat getrocknet und nach Filtration eingeengt. Das erhaltene Rohprodukt wird an Kieselgel mit Hexan/Essigsäureethylester (4:1) gereinigt.
Es werden 14,4 g (76 % der Theorie) N-(4-Brom-3-ethoxymethoxyphenyl)-carbaminsäure-tert-butylester als Öl erhalten.
¹H-NMR (300 MHz, DMSO): δ = 9,50 (s, 1 H, NH); 7,45 (d, J=2,0, 1 H, H2); 7,43 (d, J=8,6, 1 H, H5); 7,04 (dd, J=2,0, J=8,6, 1 H, H6); 5,24 (s, 2H, OCH2); 3,70 (q, J=7,1, 2H, CH2); 1,48 (s, 9H tert-butyl); 1,16 (t, J=7,1, 3H, CH3).
CHN-Analyse:

| (C₁₄H₂₀BrNO₄) | | % C | % H | % N |
|---|---|---|---|---|
| | berechnet: | 48,57 | 5,82 | 4,05 |
| | gefunden: | 47,82 | 5,87 | 3,77 |

### D. Synthese von [4-(5,5-Dimethyl-[1,3,2]dioxaborinan-2-yl)-3-ethoxymethoxy-phenyl]-carbaminsäure-tert-butylester

10 g (28,8 mmol) N-(4-Brom-3-ethoxymethoxy-phenyl)-carbaminsäure-tert-butylester aus Stufe C und 13 g (57,6 mmol) 5,5,5',5'-Tetramethyl-2,2'-Bi-[1,3,2-dioxaborinan] werden unter Argon in 260 ml Dioxan gelöst. Anschließend werden 2,11 g (2,88 mmol) [1,1'-Bis(diphenylphosphino)-ferrocen]dichlor-Palladium(II) und 8,48 g (86,4 mmol) Kaliumacetat zugegeben und die Reaktionsmischung 7 Stunden lang auf 80 °C erwärmt. Dann wird die Reaktionsmischung in 1,6 I Eis/Wasser gegossen und mit Essigsäureethylester extrahiert. Die organische Phase wird mit einer gesättigten wässrigen Kochsalz-Lösung gewaschen, über Magnesiumsulfat getrocknet und nach Filtration eingeengt. Das erhaltene Rohprodukt wird an Kieselgel mit Hexan/Essigsäureethylester (2:1) gereinigt
Es werden 5,84 g (54 % der Theorie) [4-(5,5-Dimethyl-[1,3,2]dioxaborinan-2-yl)-3-ethoxymethoxy-phenyl]-carbaminsäure-tert-butylester erhalten.
¹H-NMR (300 MHz, DMSO): δ = 9,40 (s, 1H, NH); 7,41 (d, J=8,1, 1H, H5); 7,21 (s, 1 H, H2); 7,07 (d, J=8,1, 1 H, H6); 5,09 (s, 2H, OCH2); 3,69 (s, 4H, BOCH2); 3,66 (q, J=7,1, 2H, CH2); 1,48 (s, 9H, tert-butyl); 1,18 (t, 3H, CH3); 0,95 (s, 6H, CH3).

### E. Synthese der 3-Aminophenole der Formel (I)

0,23 g (0,6 mmol) [4-(5,5-Dimethyl-[1,3,2]dioxaborinan-2-yl)-3-ethoxymethoxy-phenyl]-carbaminsäure-tert-butylester aus Stufe D und 0,78 mmol des entsprechenden Bromderivates werden unter Argon in 4 ml 1,2-Dimethoxyethan gelöst. Anschließend werden 0,07 g (0,06 mmol) Tetrakis-(triphenylphosphin)-palladium und 0,8 ml 2N Kaliumcarbonatlösung zugegeben und die Reaktionsmischung auf 80 °C erwärmt. Nach Beendigung der Reaktion wird die Reaktionsmischung in 15 ml Essigsäureethylester gegossen, die organische Phase mit einer 1N Natriumhydroxid Lösung extrahiert und sodann mit Magnesiumsulfat getrocknet. Das Lösungsmittel wird am Rotationsverdampfer abdestilliert und der Rückstand an Kieselgel mit Hexan/Essigsäureethylester gereinigt. Das so erhaltene Produkt wird in 2 ml Ethanol gelöst und mit 1 ml einer 2,9molaren ethanolischen Salzsäurelösung oder mit 4molaren Salzsäure in Dioxan versetzt. Die Reaktionsmischung wird anschließend auf 55 °C erwärmt. Nach Beendigung der Reaktion wird der Niederschlag abfiltriert, mit Ethanol bzw. Dioxan gewaschen und sodann getrocknet.
1. 5-Amino-2-(3-thienyl)phenol-hydrochlorid
Verwendetes Bromderivat: 3-Brom-thiophen
Ausbeute: 0,032 g (23% der Theorie)
ESI-MS: 190 [M-H]⁺(100)
2. 5-Amino-2-(3-furyl)phenol-hydrochlorid
Verwendetes Bromderivat: 3-Brom-furan
Ausbeute: 0,024 g (30% der Theorie)
ESI-MS: 174 [M-H]⁺ (100)
3. 5-Amino-2-(1,3-thiazol-2-yl)phenol-hydrochlorid
Verwendetes Bromderivat: 2-Brom-1,3-thiazol
Ausbeute: 0,029 g (21% der Theorie)
ESI-MS: 193 [M+H]⁺(100)
4. 5-Amino-2-(2-thienyl)phenol-hydrochlorid
Verwendetes Bromderivat: 2-Brom-thiophen
Ausbeute: 0,057 g (42% der Theorie)
ESI-MS: 190 [M-H]⁺(100)
5. 5-Amino-2-(4-methyl-3-thienyl)phenol-hydrochlorid
Verwendetes Bromderivat: 3-Brom-4-methyl-thiophen
Ausbeute: 0,038 g (26% der Theorie)
ESI-MS: 204 [M-H]⁺(100)
6. 5-Amino-2-(2-chlor-3-thienyl)phenol-hydrochlorid
Verwendetes Bromderivat: 3-Brom-2-chlor-thiophen
Ausbeute: 0,056 g (35% der Theorie)
ESI-MS: 224 [M-H]⁺ (100)
¹H-NMR (300 MHz, DMSO): δ =10,3 (s, br, 1 H, OH); 7,49 (d, J=5,7, 1 H, H5'); 7,29 (d, J=8,1, 1 H, H3); 7,10 (d, J=5,7, 1 H, H4'); 6,99 (d, J=1,8, 1 H, H6); 6,81 (dd, J=1,8, J=8,1, 1H, H4); 4,10 (s, br, 3H, NH3⁺).
CHN-Analyse:

| | | | | | |
|---|---|---|---|---|---|
| (C₁₀H₈NOSCl*0,94HCl) | % C | % H | % N | %S | %Cl |
| berechnet: | 46,20 | 3,47 | 5,39 | 12,33 | 26,46 |
| gefunden: | 46,20 | 3,70 | 5,40 | 11,80 | 26,40 |

7. 5-Amino-2-(5-chlor-2 thienyl)phenol-hydrochlorid
Verwendetes Bromderivat: 2-Brom-5-chlor-thiophen
Ausbeute: 0,066 g (58% der Theorie)
ESI-MS: 224 [M-H]⁺(100)
8. 5-Amino-2-(5-acetyl-2-thienyl)phenol-hydrochlorid
Verwendetes Bromderivat: 2-Brom-5-acetyl-thiophen
Ausbeute: 0,060 g (37% der Theorie)
ESI-MS: 256 [M+Na]⁺(100)
9. 5-Amino-2-(5-formyl-2-furyl)phenol-hydrochlorid
Verwendetes Bromderivat: 2-Brom-5-formyl-thiophen
Ausbeute: 0,050 g (33% der Theorie)
ESI-MS: 202 [M-H]⁺(100)

### Beispiele 10 bis 18: Haarfärbemittel

Es werden Haarfärbelösungen der folgenden Zusammensetzung hergestellt:

| | |
|---|---|
| 1,25 mmol | Substanz der Formel (I) gemäß Tabelle 1 |
| 1,25 mmol | Entwicklersubstanz gemäß Tabelle 1 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

10 g der vorstehenden Färbelösung werden unmittelbar vor der Anwendung mit 10 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

**Tabelle 1:**

| **Beispiel Nr.** | **Kupplersubstanz der Formel (I)** | **Entwicklersubstanz** | | | |
|---|---|---|---|---|---|
| | | **I.** | **II.** | **III.** | **IV.** |
| | | **2,5-Diamino-toluol-sulfat** | **2,5-Diamino-phenyl-ethanol-sulfat** | **4,5-Diamino-1-(2'-hydroxy-ethyl)-pyrazol-sulfat** | **4-Amino-3-methyl-phenol** |
| **10.** | gemäß Beispiel **1** | violett | violett | himbeerrot | hellrosa |
| **11.** | gemäß Beispiel **2** | hell-grauviolett | hell-grauviolett | hellrot | beige |
| **12.** | gemäß Beispiel **3** | grau | grau | pink | hellrosa |
| **13.** | gemäß Beispiel **4** | grau | grau | pink | hellrosa |
| **14.** | gemäß Beispiel **5** | hell-grauviolett | hell-grauviolett | hellrot | beige |
| **15.** | gemäß Beispiel **6** | dunkelviolett | dunkelviolett | himbeerrot | hellrosa |
| **16.** | gemäß Beispiel **7** | dunkelgrau | dunkelgrau | pink | hellrosa |
| **17.** | gemäß Beispiel **8** | grün | grün | pink | gelb |
| **18.** | gemäß Beispiel **9** | aschblond | aschblond | hellrot | beige |

### Beispiel 19 bis 24: Haarfärbemittel

Es werden Haarfärbelösungen der folgenden Zusammensetzung hergestellt:

| | |
|---|---|
| X g | 3-Aminophenol-Derivat der Formel (I) (Kupplersubstanz **K1** gemäss Tabelle 4) |
| U g | Entwicklersubstanz **E8** bis **E15** gemäß Tabelle 2 |
| Y g | Kupplersubstanz **K11** bis **K36** gemäß Tabelle 4 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

**Tabelle 2:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K1** | 5-amino-2-(2-chlor-3-thienyl)phenol |
| | |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K18** | N-(3-Dimethylamino)phenylhamstoff |
| **K19** | 1,3-Bis(2,4-diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K31** | 1,3-Dihydroxy-benzol |
| **K32** | 2-Methyl-1,3-dihydroxy-benzol. |
| **K33** | 1-Chlor-2,4-dihydroxy-benzol |
| **K34** | 4-(2'-Hydroxyethyl)amino-1,2-methylendioxybenzol*HCl |
| **K36** | 2-Amino-5-methyl-phenol |

**Tabelle 5: Haarfärbemittel**

| **Beispiel Nr.** | **19** | **20** | **21** | **22** | **23** | **24** |
|---|---|---|---|---|---|---|
| **Farbstoffe** | (Farbstoffmenge in Gramm) | | | | | |
| **K1** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **Färbeergebnis** | blond | blond | blond | blond | blond | blond |

### Beispiele 25 bis 30: Haarfärbemittel

Es werden Haarfärbelösungen der folgenden Zusammensetzung hergestellt:

| | |
|---|---|
| Xg | 3-Aminophenol-Derivat der Formel (I) (Kupplersubstanz **K1** gemäss Tabelle 4) |
| U g | Entwicklersubstanz **E8** bis **E15** gemäß Tabelle 2 |
| Y g | Kupplersubstanz **K11** bis **K36** gemäß Tabelle 4 |
| Z g | direktziehende Farbstoffe **D2** und **D3** gemäß Tabelle3 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbecreme werden unmittelbar vor der Anwendung mit 30 g einer 6-prozentigen Wasserstoffperoxidlösung vermischt. Anschließend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

**Tabelle 6: Haarfärbemittel**

| **Beispiel Nr.** | **25** | **26** | **27** | **28** | **29** | **30** |
|---|---|---|---|---|---|---|
| **Farbstoffe** | (Farbstoffmenge in Gramm) | | | | | |
| **K1** | 0,60 | 1,30 | 1,15 | 0,15 | 0,15 | 0,15 |
| **E8** | 1,50 | | | | | |
| **E11** | 0,10 | | | | | |
| **E13** | | 1,60 | | | | 0,70 |
| **E14** | | | | 0,10 | 0,10 | |
| **E15** | | | 1,80 | 0,70 | 0,70 | |
| **K12** | 0,50 | | | | | |
| **K14** | 0,10 | | | | | |
| **K18** | 0,05 | | | | | |
| **K19** | 0,10 | | | | | |
| **K23** | | | 0,05 | 0,10 | 0,10 | 0,10 |
| **K24** | 0,15 | | | | | |
| **K31** | 0,90 | 1,10 | 1,10 | 0,40 | 0,40 | 0,40 |
| **K34** | 0,10 | | | | | |
| **D2** | | | | 0,10 | 0,10 | 0,10 |
| **D3** | | | | 0,05 | 0,05 | 0,05 |
| **Färbeergebnis** | schwarz | schwarz | schwarz | braun | braun | braun |

Alle in der vorliegenden Anmeldung enthaltenen Prozentangaben stellen soweit nicht anders angegeben Gewichtsprozente dar.

## Patentansprüche

1. Mittel zur Färbung von Keratinfasern auf der Basis einer Entwicklersubstanz-Kupplersubstanz-Kombination, **dadurch gekennzeichnet, dass** es als Kupplersubstanz mindestens ein 3-Aminophenol-Derivat der Formel (I) enthält, 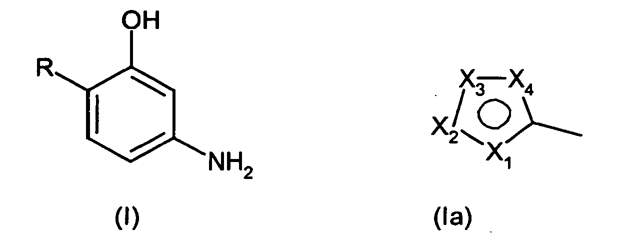 mit **R** gleich einem aromatischen, heterozyklischen 5-Ring der Formel (la);
wobei **X1, X2, X3** und **X4** unabhängig voneinander gleich Schwefel, Stickstoff, N-R1, Sauerstoff, C-R2, C-R3, C-R4 oder C-R5 sind, unter der Bedingung, dass mindestens einer und höchstens drei der Reste **X1** bis **X4** gleich Stickstoff, N-R1, Schwefel oder Sauerstoff sind und wenn es mehrere Heteroatome gibt höchstens einer der Reste **X1** bis **X4** gleich Schwefel, N-R1 oder Sauerstoff ist;
**R1** gleich Wasserstoff, einer C₁-C₆-Alkylgruppe, einer Phenylgruppe, einer (C₂-C₄)-Hydroxyalkylgruppe oder einer Acetylgruppe ist;
**R2, R3, R4 und R5** unabhängig voneinander Wasserstoff, ein Halogenatom (F, Cl, Br, J), eine Cyanogruppe, eine C₁-C₆-Alkylgruppe, eine C₁-C₄-Alkoxygruppe, eine C₁-C₄-Alkylthioethergruppe, eine Mercaptogruppe, eine Nitrogruppe, eine Aminogruppe, eine C₁-C₄-Alkylaminogruppe, eine Di(C₁-C₄)alkylaminogruppe, eine Hydroxy(C₂-C₄)alkylaminogruppe, eine Di(hydroxy(C₂-C₄)alkyl)aminogruppe, eine (Dihydroxy(C₃-C₄)alkyl)-aminogruppe, eine Trifluormethangruppe, eine -C(O)CH₃-Gruppe, eine -C(O)CF₃-Gruppe, eine -C(O)H-Gruppe, eine -Si(CH₃)₃-Gruppe, eine (C₁-C₄)-Hydroxyalkylgruppe oder eine (C₂-C₄)-Dihydroxyalkylgruppe darstellen.

2. Mittel nach Anspruch 1, **dadurch gekennzeichnet, dass** das 3-Aminophenol-Derivat der Formel (I) ausgewählt ist aus 5-amino-2-(3-thienyl)phenol, 5-amino-2-(3-furyl)phenol, 5-amino-2-(pyrrol-3-yl)phenol, 5-amino-2-(1-methyl-1H-pyrrol-3-yl)phenol, 5-amino-2-(1,3-thiazol-2-yl)phenol, 5-amino-2-(1,3-thiazol-5-yl)phenol, 5-amino-2-(2-thienyl)phenol, 5-amino-2-(2-furyl)-phenol, 5-amino-2-(pyrrol-2-yl)phenol, 5-amino-2-(1-methyl-1 H-pyrrol-2-yl)phenol, 5-amino-2-(2-chlor-3-thienyl)-phenol, 5-amino-2-(2-methyl-3-thienyl)phenol, 5-amino-2-(2-nitro-3-thienyl)phenol, 5-amino-2-(2-amino-3-thienyl)phenol, 5-amino-2-(2-acetyl-3-thienyl)-phenol, 5-amino-2-(2-formyl-3-thienyl)phenol, 5-amino-2-(4-chlor-3-thienyl)phenol, 5-amino-2-(4-methyl-3-thienyl)phenol, 5-amino-2-(4-nitro-3-thienyl)phenol, 5-amino-2-(4-amino-3-thienyl)phenol, 5-amino-2-(4-acetyl-3-thienyl)phenol, 5-amino-2-(4-formyl-3-thienyl)phenol, 5-amino-2-(5-chlor-3-thienyl)phenol, 5-amino-2-(5-methyl-3-thienyl)phenol, 5-amino-2-(5-nitro-3-thienyl)phenol, 5-amino-2-(5-acetyl-3-thienyl)phenol, 5-amino-2-(5-amino-3-thienyl)-phenol, 5-amino-2-(5-formyl-3-thienyl)phenol, 5-amino-2-(5-formyl-3-furyl)phenol, 5-amino-2-(3-chlor-2-thienyl)phenol, 5-amino-2-(3-methyl-2-thienyl)phenol, 5-amino-2-(3-nitro-2-thienyl)phenol, 5-amino-2-(3-amino-2-thienyl)phenol, 5-amino-2-(3-acetyl-2-thienyl)-phenol, 5-amino-2-(3-formyl-2-thienyl)phenol, 5-amino-2-(4-chlor-2-thienyl)phenol, 5-amino-2-(4-methyl-2-thienyl)phenol, 5-amino-2-(4-nitro-2-thienyl)phenol, 5-amino-2-(4-amino-2-thienyl)phenol, 5-amino-2-(4-acetyl-2-thienyl)phenol, 5-amino-2-(4-formyl-2-thienyl)phenol, 5-amino-2-(5-chlor-2-thienyl)phenol, 5-amino-2-(5-methyl-2-thienyl)phenol, 5-amino-2-(5-nitro-2-thienyl)phenol, 5-amino-2-(5-amino-2-thienyl)phenol, 5-amino-2-(5-acetyl-2-thienyl)-phenol, 5-amino-2-(5-formyl-2-thienyl)phenol, 5-amino-2-(5-formyl-2-furyl)phenol, 5-amino-2-(5-nitro-1,3-thiazol-2-yl)phenol, 5-amino-2-(5-amino-1,3-thiazol-2-yl)phenol, 5-amino-2-(2-nitro-1,3-thiazol-5-yl)phenol, 5-amino-2-(2-amino-1,3-thiazol-5-yl)phenol, 5-amino-2-(3,5-dimethyl-1H-pyrazol-4-yl)phenol und 5-amino-2-(5-nitro-4H-1,2,4-triazol-3-yl)phenol sowie deren physiologisch verträglichen wasserlöslichen Salzen.

3. Mittel nach Anspruch 1, **dadurch gekennzeichnet, dass** in der Formel (I) gilt: (i) **X2** ist gleich Schwefel, und **X1, X3** und **X4** sind gleich C-R2, C-R4 und C-R5 oder (ii) **X1** ist gleich Schwefel und **X2, X3** und **X4** sind gleich C-R3, C-R4 und C-R5.

4. Mittel nach einem der Ansprüche 1 bis 3, **dadurch gekennzeichnet, dass** das 3-Aminophenol-Derivat der Formel (I) ausgewählt ist aus 5-amino-2-(3-thienyl)phenol, 5-amino-2-(2-chlor-3-thienyl)phenol und 5-amino-2-(5-chlor-2-thienyl)phenol sowie deren physiologisch verträglichen Salzen.

5. Mittel nach einem der Ansprüche 1 bis 4, **dadurch gekennzeichnet, dass** das 3-Aminophenol-Derivat der Formel (I) in einer Menge von 0,005 bis 20 Gewichtsprozent enthalten ist.

6. Mittel nach einem der Ansprüche 1 bis 5, **dadurch gekennzeichnet, dass** die Entwicklersubstanz ausgewählt ist aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1 H-pyrazol, 2-Amino-phenol, 2-Amino-6-methyl-phenol, 2-Amino-5-methylphenol und 1,2,4-Trihydroxy-benzol.

7. Mittel nach einem der Ansprüche 1 bis 6, **dadurch gekennzeichnet, dass** es zusätzlich zu den Verbindungen der Formel (I) mindestens eine weitere bekannte Kupplersubstanzen enthält, welche ausgewählt ist aus der Gruppe bestehend aus N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hyd roxyphenoxy)-ethanol, 5-[(3-Hydroxypropy)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor 2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

8. Mittel nach einem der Ansprüche 1 bis 7, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

9. Mittel nach einem der Ansprüche 1 bis 8 , **dadurch gekennzeichnet, dass** es zusätzlich mindestens einen direktziehenden Farbstoff enthält.

10. Mittel nach einem der Ansprüche 1 bis 9, **dadurch gekennzeichnet, dass** es einen pH-Wert von 6,5 bis 11,5 aufweist.

11. Gebrauchsfertiges Mittel zur oxidativen Färbung von Keratinfasern, welches in einem zum Färben geeigneten Medium mindestens eine Entwicklersubstanz und mindestens eine Kupplersubstanz sowie mindestens ein Oxidationsmittel enthält, **dadurch gekennzeichnet, dass** es als Kupplersubstanz mindestens ein 3-Aminophenol-Derivat der Formel (I) nach einem der Ansprüche 1 bis 4 enthält.

12. Mittel nach einem der Ansprüche 1 bis 11, **dadurch gekennzeichnet, dass** es ein Haarfärbemittel ist.

13. 3-Aminophenol-Derivate der Formel (I), oder deren physiologisch verträglichen wasserlösliche Salze, 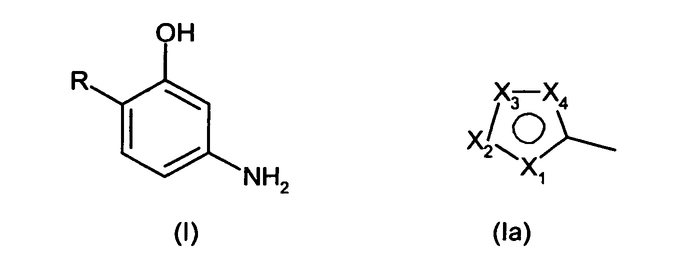 mit **R** gleich einem aromatischen, heterozyklischen 5-Ring der Formel (la);
wobei gilt: (i) **X2** ist gleich Schwefel, und **X1, X3** und **X4** sind gleich C-R2, C-R4 und C-R5 oder (ii) **X1** ist gleich Schwefel und **X2, X3** und **X4** sind gleich C-R3, C-R4 und C-R5; und
**R2, R3, R4** und **R5** stellen unabhängig voneinander Wasserstoff, ein Halogenatom (F, Cl, Br, J), eine Cyanogruppe, eine C₁-C₆-Alkylgruppe, eine C₁-C₄-Alkoxygruppe, eine C₁-C₄-Alkylthioethergruppe, eine Mercaptogruppe, eine Nitrogruppe, eine Aminogruppe, eine C₁-C₄-Alkylaminogruppe, eine Di(C₁-C₄)alkylaminogruppe, eine Hydroxy(C₂-C₄)alkylaminogruppe, eine Di(hydroxy(C₂-C₄)alkyl)aminogruppe, eine (Dihydroxy(C₃-C₄)alkyl)-aminogruppe, eine Trifluormethangruppe, eine -C(O)CH₃-Gruppe, eine -C(O)CF₃-Gruppe, eine -C(O)H-Gruppe, eine -Si(CH₃)₃-Gruppe, eine (C₁-C₄)-Hydroxyalkylgruppe oder eine (C₂-C₄)-Dihydroxyalkylgruppe dar.

14. 3-Aminophenol-Derivat, **dadurch gekennzeichnet, dass** es ausgewählt ist aus 5-amino-2-(3-thienyl)phenol, 5-amino-2-(3-furyl)phenol, 5-amino-2-(pyrrol-3-yl)phenol, 5-amino-2-(1-methyl-1H-pyrrol-3-yl)phenol, 5-amino-2-(1,3-thiazol-2-yl)phenol, 5-amino-2-(1,3-thiazol-5-yl)phenol, 5-amino-2-(2-thienyl)phenol, 5-amino-2-(2-furyl)-phenol, 5-amino-2-(pyrrol-2-yl)phenol, 5-amino-2-(1-methyl-1 H-pyrrol-2-yl)phenol, 5-amino-2-(2-chlor-3-thienyl)-phenol, 5-amino-2-(2-methyl-3-thienyl)phenol, 5-amino-2-(2-nitro-3-thienyl)phenol, 5-amino-2-(2-amino-3-thienyl)phenol, 5-amino-2-(2-acetyl-3-thienyl)-phenol, 5-amino-2-(2-formyl-3-thienyl)phenol, 5-amino-2-(4-chlor-3-thienyl)phenol, 5-amino-2-(4-methyl-3-thienyl)phenol, 5-amino-2-(4-nitro-3-thienyl)phenol, 5-amino-2-(4-amino-3-thienyl)phenol, 5-amino-2-(4-acetyl-3-thienyl)phenol, 5-amino-2-(4-formyl-3-thienyl)phenol, 5-amino-2-(5-chlor-3-thienyl)phenol, 5-amino-2-(5-methyl-3-thienyl)phenol, 5-amino-2-(5-nitro-3-thienyl)phenol, 5-amino-2-(5-acetyl-3-thienyl)phenol, 5-amino-2-(5-amino-3-thienyl)-phenol, 5-amino-2-(5-formyl-3-thienyl)phenol, 5-amino-2-(5-formyl-3-furyl)phenol, 5-amino-2-(3-chlor-2-thienyl)phenol, 5-amino-2-(3-methyl-2-thienyl)phenol, 5-amino-2-(3-nitro-2-thienyl)phenol, 5-amino-2-(3-amino-2-thienyl)phenol, 5-amino-2-(3-acetyl-2-thienyl)-phenol, 5-amino-2-(3-formyl-2-thienyl)phenol, 5-amino-2-(4-chlor-2-thienyl)phenol, 5-amino-2-(4-methyl-2-thienyl)phenol, 5-amino-2-(4-nitro-2-thienyl)phenol, 5-amino-2-(4-amino-2-thienyl)phenol, 5-amino-2-(4-acetyl-2-thienyl)phenol, 5-amino-2-(4-formyl-2-thienyl)phenol, 5-amino-2-(5-chlor-2-thienyl)phenol, 5-amino-2-(5-methyl-2-thienyl)phenol, 5-amino-2-(5-nitro-2-thienyl)phenol, 5-amino-2-(5-amino-2-thienyl)phenol, 5-amino-2-(5-acetyl-2-thienyl)-phenol, 5-amino-2-(5-formyl-2-thienyl)phenol, 5-amino-2-(5-formyl-2-furyl)phenol, 5-amino-2-(5-nitro-1,3-thiazol-2-yl)phenol, 5-amino-2-(5-amino-1,3-thiazol-2-yl)phenol, 5-amino-2-(2-nitro-1,3-thiazol-5-yl)phenol, 5-amino-2-(2-amino-1,3-thiazol-5-yl)phenol, 5-amino-2-(3,5-dimethyl-1H-pyrazol-4-yl)phenol und 5-amino-2-(5-nitro-4H-1,2,4-triazol-3-yl)phenol sowie deren physiologisch verträglichen wasserlöslichen Salzen.

15. 3-Aminophenol-Derivat nach Anspruch 13 oder 14, **dadurch gekennzeichnet, dass** es ausgewählt ist aus 5-amino-2-(3-thienyl)phenol, 5-amino-2-(2-chlor-3-thienyl)phenol und 5-amino-2-(5-chlor-2-thienyl)phenol sowie deren physiologisch verträglichen Salzen.

## Claims

1. Agent for colouring keratin fibres based on a developer substance-coupler substance combination, **characterized in that** it comprises, as coupler substance, at least one 3-aminophenol derivative of the formula (I), 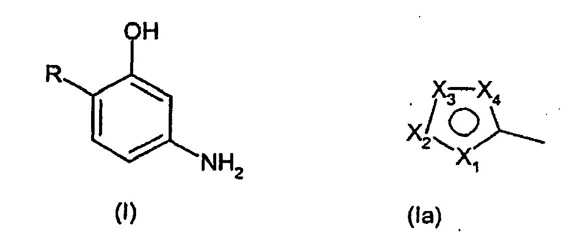 where **R** is an aromatic, heterocyclic 5-membered ring of the formula (Ia); where **X1**, **X2**, **X3** and **X4**, independently of one another, are sulphur, nitrogen, N-R1, oxygen, C-R2, C-R3, C-R4 or C-R5, with the proviso that at least one and at most three of the radicals **X1** to **X4** are nitrogen, N-R1, sulphur or oxygen and if there are two or more heteroatoms, at most one of the radicals **X1** to **X4** is sulphur, N-R1 or oxygen;
**R1** is hydrogen, a C₁-C₆-alkyl group, a phenyl group, a (C₂-C₄)-hydroxyalkyl group or an acetyl group;
**R2, R3, R4** and **R5,** independently of one another, are hydrogen, a halogen atom (F, Cl, Br, I), a cyano group, a C₁-C₆-alkyl group, a C₁-C₄-alkoxy group, a C₁-C₄-alkyl thioether group, a mercapto group, a nitro group, an amino group, a C₁-C₄-alkylamino group, a di(C₁-C₄)alkylamino group, a hydroxy(C₂-C₄)alkylamino group, a di(hydroxy(C₂-C₄)alkyl)amino group, a (dihydroxy(C₃-C₄)alkyl)amino group, a trifluoromethane group, a -C(O)CH₃ group, a -C(O)CF₃ group, a -C(O)H group, an -Si(CH₃)₃ group, a (C₁-C₄)-hydroxyalkyl group or a (C₂-C₄)-dihydroxyalkyl group.

2. Agent according to Claim 1, **characterized in that** the 3-aminophenol derivative of the formula (I) is chosen from 5-amino-2-(3-thienyl)phenol, 5-amino-2-(3-furyl)phenol, 5-amino-2-(pyrrol-3-yl)phenol, 5-amino-2-(1-methyl-1H-pyrrol-3-yl)phenol, 5-amino-2-(1,3-thiazol-2-yl)phenol, 5-amino-2-(1,3-thiazol-5-yl)phenol, 5-amino-2-(2-thienyl)phenol, 5-amino-2-(2-furyl)phenol, 5-amino-2-(pyrrol-2-yl)phenol, 5-amino-2-(1-methyl-1H-pyrrol-2-yl) phenol, 5-amino-2-(2-chloro-3-thienyl)phenol, 5-amino-2-(2-methyl-3-thienyl)phenol, 5-amino-2-(2-nitro-3-thienyl)phenol, 5-amino-2-(2-amino-3-thienyl)phenol, 5-amino-2-(2-acetyl-3-thienyl)phenol, 5-amino-2-(2-formyl-3-thienyl)phenol, 5-amino-2-(4-chloro-3-thienyl)phenol, 5-amino-2-(4-methyl-3-thienyl)phenol, 5-amino-2-(4-nitro-3-thienyl)phenol, 5-amino-2-(4-amino-3-thienyl)phenol, 5-amino-2-(4-acetyl-3-thienyl)phenol, 5-amino-2-(4-formyl-3-thienyl)phenol, 5-amino-2-(5-chloro-3-thienyl)phenol, 5-amino-2-(5-methyl-3-thienyl)phenol, 5-amino-2-(5-nitro-3-thienyl)phenol, 5-amino-2-(5-acetyl-3-thienyl)phenol, 5-amino-2-(5-amino-3-thienyl)-phenol, 5-amino-2-(5-formyl-3-thienyl)phenol, 5-amino-2-(5-formyl-3-furyl)phenol, 5-amino-2-(3-chloro-2-thienyl)phenol, 5-amino-2-(3-methyl-2-thienyl)phenol, 5-amino-2-(3-nitro-2-thienyl)phenol, 5-amino-2-(3-amino-2-thienyl)phenol, 5-amino-2-(3-acetyl-2-thienyl)phenol, 5-amino-2-(3-formyl-2-thienyl)phenol, 5-amino-2-(4-chloro-2-thienyl)phenol, 5-amino-2-(4-methyl-2-thienyl)phenol, 5-amino-2-(4-nitro-2-thienyl)-phenol, 5-amino-2-(4-amino-2-thienyl)phenol, 5-amino-2-(4-acetyl-2-thienyl)phenol, 5-amino-2-(4-formyl-2-thienyl)phenol, 5-amino-2-(5-chloro-2-thienyl)phenol, 5-amino-2-(5-methyl-2-thienyl)phenol, 5-amino-2-(5-nitro-2-thienyl)phenol, 5-amino-2-(5-amino-2-thienyl)-phenol, 5-amino-2-(5-acetyl-2-thienyl)phenol, 5-amino-2-(5-formyl-2-thienyl)phenol, 5-amino-2-(5-formyl-2-furyl)phenol, 5-amino-2-(5-nitro-1,3-thiazol-2-yl)-phenol, 5-amino-2-(5-amino-1,3-thiazol-2-yl)phenol, 5-amino-2-(2-nitro-1,3-thiazol-5-yl)phenol, 5-amino-2-(2-amino-1,3-thiazol-5-yl)phenol, 5-amino-2-(3,5-dimethyl-1H-pyrazol-4-yl)phenol and 5-amino-2-(5-nitro-4H-1,2,4-triazol-3-yl)phenol and physiologically compatible water-soluble salts thereof.

3. Agent according to Claim 1, **characterized in that**, in the formula (I): (i) **X2** is sulphur, and **X1, X3** and **X4** are C-R2, C-R4 and C-R5 or (ii) **X1** is sulphur and **X2, X3** and **X4** are C-R3, C-R4 and C-R5.

4. Agent according to one of Claims 1 to 3, **characterized in that** the 3-aminophenol derivative of the formula (I) is chosen from 5-amino-2-(3-thienyl)phenol, 5-amino-2-(2-chloro-3-thienyl)phenol and 5-amino-2-(5-chloro-2-thienyl)phenol and physiologically compatible salts thereof.

5. Agent according to one of Claims 1 to 4, **characterized in that** the 3-aminophenol derivative of the formula (I) is present in an amount of from 0.005 to 20 per cent by weight.

6. Agent according to one of Claims 1 to 5, **characterized in that** the developer substance is chosen from the group consisting of 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)amino]-aniline, 4-[di(2-hydroxyethyl)amino]aniline, 4-[di(2-hydroxyethyl)amino]-2-methylaniline, 4-[(2-methoxyethyl) amino] aniline, 4-[(3-hydroxypropyl)amino]aniline, 4-[(2,3-dihydroxypropyl)amino]aniline, 1,4-diamino-2-(1-hydroxyethyl)benzene, 1,4-diamino-2-(2-hydroxyethyl) benzene, 1,4-diamino-2-(1-methylethyl)benzene, 1,3-bis [(4-aminophenyl) (2-hydroxyethyl)amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4-5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol and 1,2,4-trihydroxybenzene.

7. Agent according to one of Claims 1 to 6, **characterized in that**, in addition to the compounds of the formula (I), it comprises at least one further known coupler substance which is chosen from the group consisting of N-(3-dimethylaminophenyl)urea, 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl) amino] aniline, 4-amino-2-di[(2-hydroxyethyl)amino]-1-ethoxybenzene, 5-methyl-2-(1-methylethyl) phenol, 3-[(2-hydroxyethyl) amino] aniline, 3- [(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylamino-phenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl) amino] phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxpyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-napthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

8. Agent according to one of Claims 1 to 7, **characterized in that** the developer substances and coupler substances, based on the total amount of the colouring agent, are each present in a total amount of from 0.005 to 20 per cent by weight.

9. Agent according to one of Claims 1 to 8, **characterized in that** it additionally comprises at least one direct dye.

10. Agent according to one of Claims 1 to 9, **characterized in that** it has a pH of from 6.5 to 11.5.

11. Ready-to-use agent for the oxidative colouring of keratin fibres, which comprises, in a medium suitable for the colouring, at least one developer substance and at least one coupler substance, and at least one oxidizing agent, **characterized in that** it comprises, as coupler substance, at least one 3-aminophenol derivative of the formula (I) according to one of Claims 1 to 4.

12. Agent according to one of Claims 1 to 11, **characterized in that** it is a hair colouring agent.

13. 3-Aminophenol derivatives of the formula (I), or physiologically compatible water-soluble salts thereof, 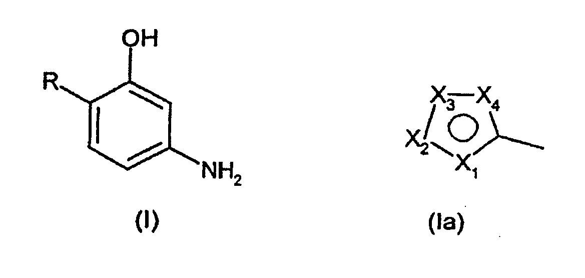 where **R** is an aromatic, heterocyclic 5-membered ring of the formula (Ia); where: (i) **X2** is sulphur, and **X1**, **X3** and **X4** are C-R2, C-R4 and C-R5 or (ii) **X1** is sulphur and **X2, X3** and **X4** are C-R3, C-R4 and C-R5; and
R2, R3, R4 and R5, independently of one another, are hydrogen, a halogen atom (F, Cl, Br, I), a cyano group, a C₁-C₆-alkyl group, a C₁-C₄-alkoxy group, a C₁-C₄-alkyl thioether group, a mercapto group, a nitro group, an amino group, a C₁-C₄-alkylamino group, a di (C₁₋C₄)alkylamino group, a hydroxy (C₂-C₄) alkylamino group, a di(hydroxy(C₂-C₄)alkyl) amino group, a (dihydroxy(C₃₋C₄)alkyl)amino group, a trifluoromethane group, a -C(O)CH₃ group, a -C(O)CF₃ group, a -C(O)H group, an -Si (CH₃)₃ group, a (C₁-C₄)-hydroxyalkyl group or a (C₂-C₄)-dihydroxyalkyl group.

14. 3-Aminophenol derivative, **characterized in that** it is chosen from 5-amino-2-(3-thienyl)phenol, 5-amino-2-(3-furyl)phenol, 5-amino-2-(pyrrol-3-yl)phenol, 5-amino-2-(1-methyl-1H-pyrrol-3-yl)phenol, 5-amino-2-(1,3-thiazol-2-yl)phenol, 5-amino-2-(1,3-thiazol-5-yl) phenol, 5-amino-2-(2-thienyl)phenol, 5-amino-2-(2-furyl)phenol, 5-amino-2-(pyrrol-2-yl) phenol, 5-amino-2-(1-methyl-1H-pyrrol-2-yl) phenol, 5-amino-2-(2-chloro-3-thienyl)phenol, 5-amino-2-(2-methyl-3-thienyl)phenol, 5-amino-2-(2-nitro-3-thienyl)phenol, 5-amino-2-(2-amino-3-thienyl)phenol, 5-amino-2-(2-acetyl-3-thienyl)phenol, 5-amino-2-(2-formyl-3-thienyl)phenol, 5-amino-2-(4-chloro-3-thienyl)phenol, 5-amino-2-(4-methyl-3-thienyl)phenol, 5-amino-2-(4-nitro-3-thienyl)phenol, 5-amino-2-(4-amino-3-thienyl)phenol, 5-amino-2-(4-acetyl-3-thienyl)phenol, 5-amino-2-(4-formyl-3-thienyl)phenol, 5-amino-2-(5-chloro-3-thienyl)phenol, 5-amino-2-(5-methyl-3-thienyl)phenol, 5-amino-2-(5-nitro-3-thienyl)phenol, 5-amino-2-(5-acetyl-3-thienyl)phenol, 5-amino-2-(5-amino-3-thienyl)phenol, 5-amino-2-(5-formyl-3-thienyl)phenol, 5-amino-2-(5-formyl-3-furyl)phenol, 5-amino-2-(3-chloro-2-thienyl)phenol, 5-amino-2-(3-methyl)-2-thienyl)phenol, 5-amino-2-(3-nitro-2-thienyl)phenol, 5-amino-2-(3-amino-2-thienyl)phenol, 5-amino-2-(3-acetyl-2-thienyl)phenol, 5-amino-2-(3-formyl-2-thienyl)phenol, 5-amino-2-(4-chloro-2-thienyl)phenol, 5-amino-2-(4-methyl-2-thienyl)phenol, 5-amino-2-(4-nitro-2-thienyl)phenol, 5-amino-2-(4-amino-2-thienyl)phenol, 5-amino-2-(4-acetyl-2-thienyl)phenol, 5-amino-2-(4-formyl-2-thienyl)phenol, 5-amino-2-(5-chloro-2-thienyl)phenol, 5-amino-2-(5-methyl-2-thienyl)phenol, 5-amino-2-(5-nitro-2-thienyl)phenol, 5-amino-2-(5-amino-2-thienyl)phenol, 5-amino-2-(5-acetyl-2-thienyl)phenol, 5-amino-2-(5-formyl-2-thienyl)phenol, 5-amino-2-(5-formyl-2-furyl)phenol, 5-amino-2-(5-nitro-1,3-thiazol-2-yl)phenol, 5-amino-2-(5-amino-1,3-thiazol-2-yl)phenol, 5-amino-2-(2-nitro-1,3-thiazol-5-yl)phenol, 5-amino-2-(2-amino-1,3-thiazol-5-yl)phenol, 5-amino-2-(3,5-dimethyl-1H-pyrazol-4-yl)phenol and 5-amino-2-(5-nitro-4H-1,2,4-triazol-3-yl)phenol, and physiologically compatible water-soluble salts thereof.

15. 3-Aminophenol derivative according to Claim 13 or 14, **characterized in that** it is chosen from 5-amino-2-(3-thienyl)phenol, 5-amino-2-(2-chloro-3-thienyl)phenol and 5-amino-2-(5-chloro-2-thienyl)phenol, and physiologically compatible salts thereof.

## Revendications

1. Composition pour la teinture de fibres de kératine, à base d'une association développeur-coupleur, **caractérisée en ce qu'**elle contient en tant que coupleur au moins un dérivé de 3-aminophénol de formule (I), 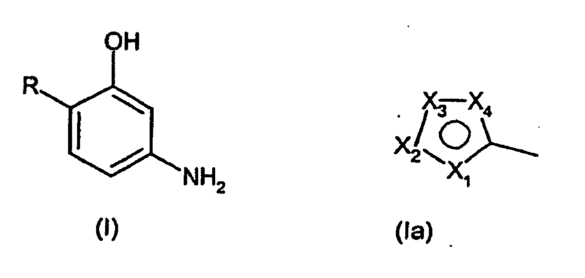 où **R** représente un cycle pentagonal aromatique hétérocyclique de formule (Ia) ;
**X1, X2, X3** et **X4** représentant, indépendamment les uns des autres, un atome de soufre, un atome d'azote, N-R1, un atome d'oxygène, C-R2, C-R3, C-R4 ou C-R5, étant entendu qu'au moins un et au maximum trois des radicaux **X1** à **X4** représente(nt) un atome d'azote, N-R1, un atome de soufre ou d'oxygène, et si plusieurs hétéroatomes sont présents un au maximum des radicaux **X1** à **X4** est un atome de soufre, N-R1 ou un atome d'oxygène ;
**R1** représente un atome d'hydrogène, un groupe alkyle en C₁-C₆, un groupe phényle, un groupe hydroxyalkyle en C₂-C₄ ou un groupe acétyle ;
**R2, R3, R4** et **R5** représentent, indépendamment les uns des autres, un atome d'hydrogène, un atome d'halogène (F, Cl, Br, I), un groupe cyano, un groupe alkyle en C₁-C₆, un groupe alcoxy en C₁-C₄, un groupe alkyl(C₁-C₄)thioéther, un groupe mercapto, un groupe nitro, un groupe amino, un groupe alkyl(C₁-C₄)amino, un groupe dialkyl(C₁-C₄)- amino, un groupe hydroxyalkyl (C₂-C₄) amino, un groupe di [hydroxyalkyl (C₂-C₄) amino, un groupe [dihydroxyalkyl(C₃-C₄)]amino, un groupe trifluorométhane, un groupe -C(O)CH₃, un groupe -C(O)CF₃, un groupe -C(O)H, un groupe -Si(CH₃)₃, un groupe hydroxyalkyle en C₁-C₄ ou un groupe dihydroxyalkyle en C₂-C₄.

2. Composition selon la revendication 1, **caractérisée en ce que** le dérivé de 3-aminophénol de formule (I) est choisi parmi le 5-amino-2-(3-thiényl)phénol, le 5-amino-2-(3-furyl)phénol, le 5-amino-2-(pyrrol-3-yl)phénol, le 5-amino-2-(1-méthyl-1H-pyrrol-3-yl)phénol, le 5-amino-2-(1,3-thiazol-2-yl)phénol, le 5-amino-2-(1,3-thiazol-5-yl)phénol, le 5-amino-2-(2-thiényl)phénol, le 5-amino-2-(2-furyl)phénol, le 5-amino-2-(pyrrol-2-yl)phénol, le 5-amino-2-(1-méthyl-1H-pyrrol-2-yl)phénol, le 5-amino-2-(2-chloro-3-thiényl)-phénol, le 5-amino-2-(2-méthyl-3-thiényl)phénol, le 5-amino-2-(2-nitro-3-thiényl)phénol, le 5-amino-2-(2-amino-3-thiényl)phénol, le 5-amino-2-(2-acétyl-3-thiényl)phénol, le 5-amino-2-(2-formyl-3-thiényl)phénol, le 5-amino-2-(4-chloro-3-thiényl)phénol, le 5-amino-2-(4-méthyl-3-thiényl)-phénol, le 5-amino-2-(4-nitro-3-thiényl)phénol, le 5-amino-2-(4-amino-3-thiényl)phénol, le 5-amino-2-(4-acétyl-3-thiényl)phénol, le 5-amino-2-(4-formyl-3-thiényl)phénol, le 5-amino-2-(5-chloro-3-thiényl)phénol, le 5-amino-2-(5-méthyl-3-thiényl)-phénol, le 5-amino-2-(5-nitro-3-thiényl)phénol, le 5-amino-2-(5-acétyl-3-thiényl)phénol, le 5-amino-2-(5-amino-3-thiényl)phénol, le 5-amino-2-(5-formyl-3-thiényl)phénol, le 5-amino-2-(5-formyl-3-furyl)phénol, le 5-amino-2-(3-chloro-2-thiényl)-phénol, le 5-amino-2-(3-méthyl-2-thiényl)phénol, le 5-amino-2-(3-nitro-2-thiényl)phénol, le 5-amino-2-(3-amino-2-thiényl)phénol, le 5-amino-2-(3-acétyl-2-thiényl)phénol, le 5-amino-2-(3-formyl-2-thiényl)phénol, le 5-amino-2-(4-chloro-2-thiényl)phénol, le 5-amino-2-(4-méthyl-2-thiényl)-phénol, le 5-amino-2-(4-nitro-2-thiényl)phénol, le 5-amino-2-(4-amino-2-thiényl)phénol, le 5-amino-2-(4-acétyl-2-thiényl)phénol, le 5-amino-2-(4-formyl-2-thiényl)phénol, le 5-amino-2-(5-chloro-2-thiényl)phénol, le 5-amino-2-(5-méthyl-2-thiényl)-phénol, le 5-amino-2-(5-nitro-2-thiényl)phénol, le 5-amino-2-(5-amino-2-thiényl)phénol, le 5-amino-2-(5-acétyl-2-thiényl)phénol, le 5-amino-2-(5-formyl-2-thiényl)phénol, le 5-amino-2-(5-formyl-2-furyl)phénol, le 5-amino-2-(5-nitro-1,3-thiazol-2-yl)phénol, le 5-amino-2-(5-amino-1,3-thiazol-2-yl)phénol, le 5-amino-2-(2-nitro-1,3-thiazol-5-yl)phénol, le 5-amino-2-(2-amino-1,3-thiazol-5-yl)phénol, le 5-amino-2-(3,5-diméthyl-1H-pyrazol-4-yl)phénol et le 5-amino-2-(5-nitro-4H-1,2,4-triazol-3-yl)phénol, ainsi que leurs sels physiologiquement acceptables.

3. Composition selon la revendication 1, **caractérisée en ce que** dans la formule (I) les symboles ont les significations suivantes : (i) **X2** représente un atome de soufre et **X1, X3** et **X4** représentent C-R2, C-R4 et C-R5 ou (ii) **X1** est un atome de soufre et **X2, X3** et **X4** représentent C-R3, C-R4 et C-R5.

4. Composition selon l'une quelconque des revendications 1 à 3, **caractérisée en ce que** le dérivé de 3-aminophénol de formule (I) est choisi parmi le 5-amino-2-(3-thiényl)phénol, le 5-amino-2-(2-chloro-3-thiényl)phénol et le 5-amino-2-(5-chloro-2-thiényl)phénol ainsi que leurs sels physiologiquement acceptables

5. Composition selon l'une quelconque des revendications 1 à 4, **caractérisée en ce que** le dérivé de 3-aminophénol de formule (I) est contenu en une quantité totale de 0,005 à 20 % en poids.

6. Composition selon l'une quelconque des revendications 1 à 5, **caractérisée en ce que** le développeur est choisi dans le groupe constitué par le 1,4-diaminobenzène, le 1,4-diamino-2-méthylbenzène, le 1,4-diamino-2,6-diméthylbenzène, le 1,4-diamino-3,5-diéthylbenzène, le 1,4-diamino-2,5-diméthylbenzène, le 1,4-diamino-2,3-diméthylbenzène, le 2-chloro-1,4-diaminobenzène, le 1,4-diamino-2-(thiophén-2-yl)benzène, le 1,4-diamino-2-(thiophén-3-yl)benzène, le 1,4-diamino-2-(pyridin-3-yl)benzène, le 2,5-diaminobiphényle, le 1,4-diamino-2-méthoxyméthylbenzène, le 1,4-diamino-2-aminométhylbenzène, le 1,4-diamino-2-hydroxyméthylbenzène, le 1,4-diamino-2-(2-hydroxyéthoxy)benzène, le 2-(2-(acétylamino)-éthoxy)-1,4-diaminobenzène, la 4-phénylamino-aniline, la 4-diméthylamino-aniline, la 4-diéthylamino-aniline, la 4-dipropylamino-aniline, la 4-[éthyl-(2-hydroxyéthyl)amino]aniline, la 4-[di(2-hydroxyéthyl)amino]aniline, la 4-[di(2-hydroxyéthyl)amino]-2-méthylaniline, la 4-[(2-méthoxyéthyl)amino]aniline, la 4-[(3-hydroxypropyl)amino]aniline, la 4-[(2,3-dihydroxypropyl)amino]aniline, le 1,4-diamino-2-(1-hydroxyéthyl) benzène, le 1,4-diamino-2-(2-hydroxyéthyl) benzène, le 1,4-diamino-2-(1-méthyléthyl)-benzène, le 1,3-bis[(4-aminophényl)(2-hydroxyéthyl)amino]-2-propanol, le 1,4-bis[(4-aminophényl)amino]butane, le 1,8-bis (2, 5-diaminophénoxy)-3,6-dioxaoctane, le 4-aminophénol, le 4-amino-3-méthylphénol, le 4-amino-3-(hydroxyméthyl) phénol, le 4-amino-3-fluorophénol, le 4-méthylaminophénol, le 4-amino-2-(aminométhyl)-phénol, le 4-amino-2-(hydroxyméthyl)phénol, le 4-amino-2-fluorophénol, le 4-amino-2-[(2-hydroxyéthyl)amino]méthylphénol, le 4-amino-2-méthylphénol, le 4-amino-2-(méthoxyméthyl)phénol, le 4-amino-2-(2-hydroxyéthyl)phénol, l'acide 5-aminosalicylique, la 2,5-diaminopyridine, la 2,4,5,6-tétraaminopyrimidine, la 2,5,6-triamino-4(1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 1-[(4-chlorophényl)méthyl]-4,5-diamino-1H-pyrazole, le 4,5-diamino-1-méthyl-1H-pyrazole, le 2-aminophénol, le 2-amino-6-méthylphénol, le 2-amino-5-méthylphénol et le 1,2,4-trihydroxybenzène.

7. Composition selon l'une quelconque des revendications 1 à 6, **caractérisée en ce qu'**elle contient, en plus des composés de formule (I), au moins un autre coupleur connu, qui est choisi dans le groupe constitué par la N-(3-diméthylaminophényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzole, le 2,4-diamino-1-(2-hydroxyéthoxy)benzène, le 1,3-diamino-4-(2,3-dihydroxypropoxy)benzène, le 1,3-diamino-4-(3-hydroxypropoxy)benzène, le 1,3-diamino-4-(2-méthoxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)-amino]aniline, la 3-[(2-aminoéthyl)amino]aniline, le 1,3-di(2,4-diaminophénoxy)propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)-amino]-4-méthoxy-2-méthylphénol, le 5-[(2-hydroxyéthyl)amino]-2-méthylphénol, le 3-[(2-hydroxyéthyl)amino]phénol, le 3-[(2-méthoxyéthyl)-amino]phénol, le 5-amino-2-éthylphénol, le 5-amino-2-méthoxyphénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, la 2,6-dihydroxy-3,4-diméthylpyridine, le 5-amino-4-chloro-2-méthylphénol, le 1-naphtol, le 2-méthyl-1-naptol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

8. Composition selon l'une quelconque des revendications 1 à 7, **caractérisée en ce que** les développeurs et coupleurs sont contenus chacun, par rapport à la quantité totale de la composition de teinture, en une quantité totale de 0,005 à 20 % en poids.

9. Composition selon l'une quelconque des revendications 1 à 8, **caractérisée en ce qu'**en outre elle contient au moins un colorant direct.

10. Composition selon l'une quelconque des revendication 1 à 9, **caractérisée en ce qu'**elle présente un pH de 6,5 à 11,5.

11. Composition prête à l'emploi, pour la teinture par oxydation de fibres de kératine, qui contient, dans un milieu approprié à la teinture, au moins un développeur et au moins un coupleur ainsi qu'au moins un oxydant, **caractérisée en ce qu'**elle contient en tant que coupleur au moins un dérivé de 3-aminophénol de formule (I) selon l'une quelconque des revendications 1 à 4.

12. Composition selon l'une quelconque des revendications 1 à 11, **caractérisée en ce qu'**il s'agit d'une composition de teinture pour cheveux.

13. Dérivés de 3-aminophénol de formule (I) ou sels physiologiquement acceptables de tels dérivés, 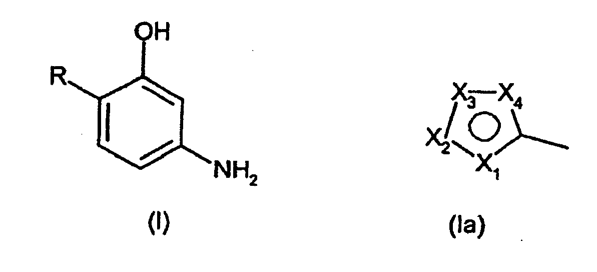 où **R** représente un cycle pentagonal aromatique hétérocyclique de formule (Ia) ;
(i) **X2** étant un atome de soufre et **X1, X3** et **X4** représentant C-R2, C-R4 et C-R5, ou (ii) **X1** étant un atome de soufre et **X2, X3** et **X4** représentant C-R3, C-R4 et C-R5 et
**R2, R3, R4** et **R5** représentent, indépendamment les uns des autres, un atome d'hydrogène, un atome d'halogène (F, Cl, Br, I), un groupe cyano, un groupe alkyle en C₁-C₆, un groupe alcoxy en C₁-C₄, un groupe alkyl(C₁-C₄)thioéther, un groupe mercapto, un groupe nitro, un groupe amino, un groupe alkyl(C₁-C₄) amino, un groupe dialkyl(C₁-C₄) amino, un groupe hydroxyalkyl-(C₂-C₄) amino, un groupe di[hydroxyalkyl(C₂-C₄)]-amino, un groupe [dihydroxyalkyl(C₃-C₄)]amino, un groupe trifluorométhane, un groupe -C(O)CH₃, un groupe -C(O)CF₃, un groupe -C(O)H, un groupe -Si(CH₃)₃, un groupe hydroxyalkyle en C₁-C₄ ou un groupe dihydroxyalkyle en C₂-C₄.

14. Dérivé de 3-aminophénol, **caractérisé en ce qu'**il est choisi parmi le 5-amino-2-(3-thiényl)phénol, le 5-amino-2-(3-furyl)phénol, le 5-amino-2-(pyrrol-3-yl)phénol, le 5-amino-2-(1-méthyl-1H-pyrrol-3-yl)phénol, le 5-amino-2-(1,3-thiazol-2-yl)phénol, le 5-amino-2-(1,3-thiazol-5-yl)phénol, le 5-amino-2-(2-thiényl)phénol, le 5-amino-2-(2-furyl)phénol, le 5-amino-2-(pyrrol-2-yl)phénol, le 5-amino-2-(1-méthyl-1H-pyrrol-2-yl)phénol, le 5-amino-2-(2-chloro-3-thiényl)phénol, le 5-amino-2-(2-méthyl-3-thiényl)phénol, le 5-amino-2-(2-nitro-3-thiényl)phénol, le 5-amino-2-(2-amino-3-thiényl)phénol, le 5-amino-2-(2-acétyl-3-thiényl)phénol, le 5-amino-2-(2-formyl-3-thiényl)phénol, le 5-amino-2-(4-chloro-3-thiényl)phénol, le 5-amino-2-(4-méthyl-3-thiényl)-phénol, le 5-amino-2-(4-nitro-3-thiényl)phénol, le 5-amino-2-(4-amino-3-thiényl)phénol, le 5-amino-2-(4-acétyl-3-thiényl)phénol, le 5-amino-2-(4-formyl-3-thiényl)phénol, le 5-amino-2-(5-chloro-3-thiényl)phénol, le 5-amino-2-(5-méthyl-3-thiényl)-phénol, le 5-amino-2-(5-nitro-3-thiényl)phénol, le 5-amino-2-(5-acétyl-3-thiényl)phénol, le 5-amino-2-(5-amino-3-thiényl)phénol, le 5-amino-2-(5-formyl-3-thiényl) phénol, le 5-amino-2-(5-formyl-3-furyl) phénol, le 5-amino-2-(3-chloro-2-thiényl)-phénol, le 5-amino-2-(3-méthyl-2-thiényl)phénol, le 5-amino-2-(3-nitro-2-thiényl)phénol, le 5-amino-2-(3-amino-2-thiényl)phénol, le 5-amino-2-(3-acétyl-2-thiényl)phénol, le 5-amino-2-(3-formyl-2-thiényl)phénol, le 5-amino-2-(4-chloro-2-thiényl)phénol, le 5-amino-2-(4-méthyl-2-thiényl)-phénol, le 5-amino-2-(4-nitro-2-thiényl)phénol, le 5-amino-2-(4-amino-2-thiényl)phénol, le 5-amino-2-(4-acétyl-2-thiényl)phénol, le 5-amino-2-(4-formyl-2-thiényl)phénol, le 5-amino-2-(5-chloro-2-thiényl)phénol, le 5-amino-2-(5-méthyl-2-thiényl)-phénol, le 5-amino-2-(5-nitro-2-thiényl)phénol, le 5-amino-2-(5-amino-2-thiényl)phénol, le 5-amino-2-(5-acétyl-2-thiényl)phénol, le 5-amino-2-(5-formyl-2-thiényl)phénol, le 5-amino-2-(5-formyl-2-furyl)phénol, le 5-amino-2-(5-nitro-1,3-thiazol-2-yl)phénol, le 5-amino-2-(5-amino-1,3-thiazol-2-yl)phénol, le 5-amino-2-(2-nitro-1,3-thiazol-5-yl)phénol, le 5-amino-2-(2-amino-1,3-thiazol-5-yl)phénol, le 5-amino-2-(3,5-diméthyl-1H-pyrazol-4-yl)phénol et le 5-amino-2-(5-nitro-4H-1,2,4-triazol-3-yl)phénol, ainsi que leurs sels physiologiquement acceptables.

15. Dérivé de 3-aminophénol selon la revendication 13 ou 14, **caractérisé en ce qu'**il est choisi parmi le 5-amino-2-(3-thiényl)phénol, le 5-amino-2-(2-chloro-3-thiényl)phénol et le 5-amino-2-(5-chloro-2-thiényl)phénol ainsi que leurs sels physiologiquement acceptables
